# EUROPEAN PATENT SPECIFICATION

(11) **EP 3 173 076 B1**
(45) Date of publication and mention of the grant of the patent: **14.07.2021**
(21) Application number: 16020180.2
(22) Date of filing: 14.05.2016
(51) Int. Cl.: A61K 9/20, A61K 31/245, A61K 31/80

(54) **A PHARMACEUTICAL COMPOSITION COMPRISING SIMETHICONE AND OTILONIUM**
PHARMAZEUTISCHE ZUSAMMENSETZUNG MIT SIMETHICON UND OTILONIUM
COMPOSITION PHARMACEUTIQUE COMPRENANT DE LA SIMÉTHICONE ET DE L'OTILONIUM

(30) Priority: 14.05.2015 TR 201505845
(43) Date of publication of application: 31.05.2017
(73) Proprietor: Abdi Ibrahim Ilac Sanayi ve Ticaret A.S., 34500 Esenyurt/Istanbul (TR)
(72) Inventor: Farsi, Ferhad, 34500 Esenyurt/Istanbul (TR); Dude, Udaya Kumar, 34500 Esenyurt/Istanbul (TR); Bayrak, Gokhan, 34500 Esenyurt/Istanbul (TR); Akdag, Kadriye, 34500 Esenyurt/Istanbul (TR)

(56) References cited:
- WO-A1-2010/092436
- WO-A1-2016/200345
- CN-A- 104 523 717
- Anonymous: "SIMFLAT Film Tablet (Product Information)", , 19 February 2014 (2014-02-19), pages 1-6, XP055356467, internet Retrieved from the Internet: URL:http://www.guncel.tgv.org.tr/simflat.h tml [retrieved on 2017-03-20]
- Mehmet Vedat Egilmez ET AL: "Patent Application No. TR 2013/01717 (Pharmaceutical combinations of simethicone and otilonium)", Internet, Turkish Patent Office, 21 August 2014 (2014-08-21), pages 1-1, XP055356425, Retrieved from the Internet: URL:http://online.turkpatent.gov.tr/EPATEN T/servlet/EPreSearchRequestManager [retrieved on 2017-03-20] & Mehmet Vedat Egilmez ET AL: "Patent Application No. TR 2013/01717 (Pharmaceutical combinations of simethicone and otilonium)", Internet, Turkish Patent Office, 21 August 2014 (2014-08-21), pages 1-17, XP055356426, Retrieved from the Internet: URL:http://online.turkpatent.gov.tr/EPATEN T/servlet/EPreSearchRequestManager [retrieved on 2017-03-20]
- Mehmet Vedat Egilmez ET AL: "Patent Application No. TR 2013/00014 (Pharmaceutical combinations of simethicone and otilonium)", Internet, Turkish Patent Office, 21 July 2014 (2014-07-21), pages 1-1, XP055356411, Retrieved from the Internet: URL:http://online.turkpatent.gov.tr/EPATEN T/servlet/EPreSearchRequestManager [retrieved on 2017-03-20] & Mehmet Vedat Egilmez ET AL: "Patent Application No. TR 2013/00014 (Pharmaceutical combinations of simethicone and otilonium)", Internet, Turkish Patent Office, 21 July 2014 (2014-07-21), pages 1-8, XP055356416, Retrieved from the Internet: URL:http://online.turkpatent.gov.tr/EPATEN T/servlet/EPreSearchRequestManager [retrieved on 2017-03-20]
- None

## Description

### Technical Field of the Invention

The invention relates to solid form pharmaceutical compositions comprising simethicone and otilonium as drug substances and the use of such compositions in the treatment of irritable bowel syndrome (IBS) and/or pain and spasm of the distal enteric tract.

### Background of the Invention

Otilonium bromide is an agent with a strong anti-spastic effect on the smooth muscles of the digestive tract. It is used as an antispasmodic for treating spastic painful conditions of the distal tract of the intestinal tract, including irritable bowel syndrome. It has a complex mechanism of action. Otilonium bromide may alter the Ca²⁺ flow from the main intracellular and extracellular regions and inhibits Ca²⁺ from entering the smooth muscle cells by binding to the muscarinic and tachykinin receptors of Ca²⁺ channels. The activity of Otilonium bromide may be described as a combination of the blockage of the Ca²⁺ channel and the mild antimuscarinic effect.

Its chemical name is N,N-Diethyl-N-methyl-2-(4-[2-(octyloxy)benzamido] benzoyloxy) ethanaminium and its structure is as shown in Formula I.

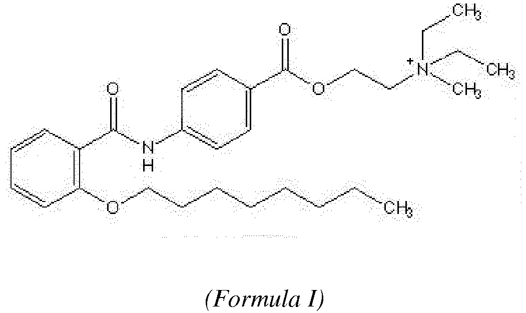

Simethicone is a drug used to relieve the pain and flatulence (pressure) originated from too much gas that presents in the digestive tract. It relieves flatulence by dispelling the gas bubbles in the gastrointestinal tract by reducing the surface tension of gas bubbles and by preventing the formation of mucous-covered gas bubbles. It is almost never absorbed by the body and is not miscible with the bloodstream.

Simethicone is chemically expressed as poly(dimethylsiloxane), silicon dioxide and its chemical structure is as shown in Formula II.

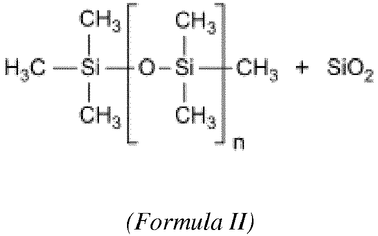

Simethicone is a white-grey, semi-transparent, oil-like viscous liquid. It is insoluble in water and has sticky properties. Special process methods should be performed to formulate simethicone as a solid dosage form due to its liquid and sticky properties. Free powder viscosity and high compressibility are desired and sought-after characteristics particularly in large-scale production of solid dosage forms in particular. In the state of the art, liquid simethicone and a solid carrier are formulated together in a solid dosage form by using several techniques.

International (PCT) Publication No. WO2008056200 relates to the compositions containing simethicone, silicate salt and adsorbent. The silicate salt used in the composition may be selected from the group consisting of calcium silicate, magnesium trisilicate, aluminum silicate, magnesium aluminum silicate and adsorbent; cellulose, microcrystalline cellulose, silicified microcrystalline cellulose, tribasic calcium phosphate, dibasic calcium phosphate, maltodextrin, precipitated silica, colloidal silica, starch, pregelatinized starch, hydroxypropyl cellulose, magnesium oxide and magnesium hydroxide. In this application, another adsorbent was also used in addition to the basic silica salt.

European Patent No. EP0891776B1 describes a freely viscous granule composition containing 10% of simethicone by weight, granular anhydrous tribasic or dibasic calcium phosphate and silicon dioxide or powdered anhydrous calcium phosphate. Within the scope of the patent, basic calcium phosphate salt is used in the composition.

U.S.Patent No. US4906478 relates to gas-relieving compositions containing 40-60% of basic calcium silicate and 60-40% of simethicone.

U.S. Patent No. US7341742B describes a pharmaceutical composition comprising a mixture of granular simethicone and basic magnesium carbonate. In the aforesaid composition, the ratio of magnesium carbonate to granular simethicone is 90:125 to 115:125. In granular simethicone, liquid simethicone is adsorbed onto maltodextrin particles in accordance with patent no. EP0425450B1.

U.S.Patent No. US5458886 described granules comprising of a combination of free flowing titanium dioxide and simethicone. The basic titanium dioxide used in the formulation has a specific particle size and surface area.

European Patent No. EP1297825B1 describes the formulation containing simethicone and microcrystalline cellulose and basic magnesium aluminometasilicate as adsorbent. The simethicone: adsorbent ratio in the formulation is 1:2.22.

European Patent Application No. EP1235559 relates to solid oral dosage forms containing a solid carrier such as basic magnesium aluminometasilicate or dibasic calcium phosphate and simethicone.

International (PCT) Publication No. WO2013095111 relates to a formulation containing 10-60% of simethicone, 20-70% of basic calcium phosphate and 20-70% of mannitol.

European Patent No.EP0194202B1 describes a pharmaceutical composition containing simethicone, 0.2 g of guar gum, 0.5 g of magnesium hydroxide, 0.5 g of aluminum hydroxide and 0.3 g of aluminum phosphate.

In the state of the art, simethicone is adsorbed or granulated with a basic agent in the compositions containing simethicone. The adsorption or granulation of simethicone with a basic agent improves free flowing characteristic and provides processability of the simethicone in the process steps to obtain a solid dosage form.

It is well-known that combining simethicone with an antidiarrheal, anti-peristaltic, anti-spastic agent or an H2 blocker is an efficient treatment option for the treatment of gastrointestinal conditions such irritable bowel syndrome (IBS) and/or the spastic-painful conditions of the distal enteric tract. Patent document EP0428296B1 describes the combination of simethicone with an antidiarrheal agent selected from the group consisting of loperamide, attapulgite, diphenoxylate, polycarbophil, calcium carbophil.

Otilonium is an anti-spastic agent and using of it with simethicone may prove to be a favorable option for individuals requiring combination therapy However, the inventors have experienced some unforeseen difficulties during formulating simethicone and otilonium together. It was already above mentioned that simethicone is hard to be formulated as a solid dosage form due to its viscous liquid like oil properties. It was also observed in the art that the basic agents which are used to facilitate the formulation of simethicone as a solid dosage form have a negative effect on otilonium stability and cause increases in the amount of impurities in the formulation. Table 1 is directed to the stability studies of simethicone and otilonium composition which is formulated by using a basic agent Tricalcium phosphate is used as basic agent in the aforesaid composition and the detailed unit formula is as follows:

| **Ingredients** | **Unit Formula (mg/tb.)** |
|---|---|
| **Drug Substance** | |
| Simethicone LV EP | 80 |

| **Excipients** | |
|---|---|
| Tricalcium phosphate | 130 |
| Calcium carbonate | 145 |
| Microcrystalline cellulose Type 101 | 45 |
| Lactose granules | 42 |
| Colloidal silicon dioxide (Aerosil 200) | 5 |
| Magnesium stearate | 3 |
| ***1. Layer Core Tablet Weight*** | **450** |

| **Drug Substance** | |
|---|---|
| Otilonium bromide | 40 |

| **Excipients** | |
|---|---|
| Lactose granules | 167.5 |
| Pregelatinized corn starch | 35 |
| Sodium starch glycolate | 5 |
| Kollidon VA 64 | 5 |
| Colloidal silicon dioxide (Aerosil 200) | 0.5 |
| Magnesium stearate | 2 |
| ***2. Layer Core Tablet Weight*** | **255** |
| ***Bilayer Core Tablet Weight*** | **705** |
| Opadry AMB Yellow 80W22002 | 15 |
| Purified Water | q.s |
| ***Bilayer Film Tablet Weight*** | **720** |

Furthermore, it is known that simethicone reduces the dissolution rate and absorption of drug substances which is combined with it. Patent document EP0571217B1 describes a separative barrier to prevent negative effect of simethicone on the dissolution rate when simethicone and other antidiarrheal, anti-peristaltic or H2 blocker agents are combined. According to the patent document, the composition is comprised of one part containing cimetidine, ranitidine, famotidine, diphenoxylate, loperamide, loperamide-N-oxide and another part containing simethicone. These parts are separated by a simethicone-impermeable barrier or a simethicone-impermeable coating is applied on the drug substances to separate simethicone of them. Thus, physical contact of simethicone and other drug substances in the composition are prevented. However, using of a simethicone-impermeable barrier or coating in the composition causes more complex production process more complex and increasing costs.

There is need to develop stable solid dosage forms comprising simethicone and otilonium with a suitable dissolution profile.

The inventors have surprisingly formulated simethicone as a solid dosage form without using a basic agent, so stability problem based on using otilonium with basic agent has been removed. Moreover, the dissolution rate and absorption of both simethicone and otilonium were also prevented from being decreased by this invention formulation. The presentation of simethicone and otilonium in the formulation exhibits the desired dissolution and absorption profiles and remains stable throughout the shelf life.

The advantages of the formulations of the present invention are as follows;
- In contrast to the compositions currently available in the art, this formulation does not contain any basic agents and thus, otilonium and the finished dosage form remain stable during the shelf life
- Simethicone that is a viscous liquid like oil become suitable for use in solid dosage forms without using any basic agents, and possesses the desired free flow and compressibility characteristics
- Decreasing of dissolution rate and absorption of otilonium is prevented based on simethicone
- A simple production method is performed and the composition does not contain any simethicone impermabl barrier or otilonium is coated by simethicone-impermeable coating
- The cost is decreased due to simple production method and not using excipients which are used in complex production methods.

### Description of the Invention

The present invention relates to a pharmaceutical composition in the form of a bilayer tablet comprising of a first part containing simethicone absorbed on colloidal silicon dioxide and a second part containing otilonium or pharmaceutically acceptable salts thereof, wherein the simethicone is absorbed on colloidal silicone dioxide and wherein the composition does not contain any basic agent.

In the object of the invention, simethicone is absorbed on colloidal silicon dioxide. Thus, it was surprisingly found that negative effect of the basic agent on the stability of otilonium in the composition is removed, and simethicone's free flow characteristic is improved and provides processability of the simethicone which are need to manufacture a solid dosage form. Stability studies of the present invention are presented in Table-2. 6-month stability studies conducted at 25°C/60% RH (Relative Humidity), 30°C/65% RH and 40°C/75% RH explicitly demonstrate that the composition is stable.

Another object of the present invention, simethicone absorbed on colloidal silicon dioxide is then mixed with either microcrystalline cellulose or silicified microcrystalline cellulose. In this way, the desired and required free flow and compressibility properties of simethicone is improved during the production process. In addition to cost reduction due to avoidance of using basic agent, simplifying production techniques is the additional advantages of the formulation.

According to this embodiment, the composition comprises simethicone, otilonium, colloidal silicon dioxide and microcrystalline cellulose or silicified microcrystalline cellulose.

In another object of the invention, the amount of simethicone in the composition is between 5-25% by weight based on the total weight of the composition. The composition of the present invention comprises simethicone in the range of 10-1000 mg.

In further object of the invention, the amount of otilonium in the composition is between 1-10% by weight based on the total weight of the composition. Otilonium in the present invention may be a pharmaceutically acceptable salt, hydrate, solvate, ester, crystal form, amorphous form, enantiomer and/or derivatives thereof. The composition of the present invention preferably comprise otilonium bromide. The composition of the present invention comprise otilonium in an amount of between 10-500 mg or equivalent otilonium bromide.

In another object of the invention, the composition comprises at least one pharmaceutically acceptable excipient in addition to simethicone, otilonium, colloidal silicon dioxide and microcrystalline cellulose or silicified microcrystalline cellulose.

In another object invention, excipients may be selected from the group consisting of fillers, binders, disintegrants, glidants and lubricants.

The filler to be used in the composition of the present invention may be selected from the group consisting of microcrystalline cellulose, lactose, maltose, mannitol and sugar. The preferred filler is lactose.

The binder to be used in the composition of the present invention may be selected from the group consisting of polyvinylpyrrolidone, copovidone, cellulose, ethyl cellulose, hydroxymethyl cellulose, hydroxypropyl methyl cellulose, lactose, maltose and mannitol. The preferred binder is copovidone.

The disintegrant to be used in the composition of the present invention may be selected from the group consisting of carboxymethyl cellulose, hydroxypropyl cellulose, hydroxypropyl methylcellulose, polyvinylpyrrolidone, crospovidone, microcrystalline cellulose, sodium starch glycolate, alginic acid and sodium alginate. The preferred disintegrant is crospovidone.

The glidant to be used in the formulation of the present invention may be selected from the group consisting of magnesium stearate, colloidal silicon dioxide, starch and talc.

The lubricant to be used in the composition of the present invention may be selected from the group consisting of stearic acid, magnesium stearate, talc, polyethylene glycol, sodium stearyl fumarate and sodium lauryl sulfate.

In order to demonstrate the effect of simethicone on the dissolution profile of otilonium bromide, the inventors performed dissolution studies with pH 4.5 acetate buffer. In this studies, 40 mg of otilonium bromide was taken to a dissolution vessel and mixed for 15 minutes at 500 rpm. A sample was collected and analyzed. Then 80 mg of simethicone was added to the dissolution vessel and mixed for 15 minutes at 500 rpm. Samples were collected at 30^{th} minute, 60^{th} minute and 90^{th} minute, and analyzed. The amount of otilonium bromide on the basis of assay analysis is given in Table 3.

As clearly seen from values given in Table-4, otilonium's dissolution was decreased by about 20% after the addition of simethicone. In order to prevent this decrease in dissolution, the inventors compressed the formulation of the present invention as a bilayer tablet comprising of the first part containing simethicone and the second part containing otilonium.

The composition comprises two parts (portions). However, differently from the art, there are no simethicone-impermeable barriers between these two parts and/or otilonium is not coated with a simethicone-impermeable coating. For this reason, it is possible to manufacture the composition of the present invention more easily by using simple and more cost-efficient methods than the current of the art.

The formulation of the present invention preferably consists of the following;
Part 1; Simethicone; colloidal silicon dioxide, microcrystalline cellulose or silicified microcrystalline cellulose, binder, glidant, lubricant;
Part 2 is formed from otilonium, filler, disintegrant, binder, glidant, lubricant.

In the composition of the present invention, after being absorbed on colloidal silicon dioxide, simethicone is combined with the other excipients in Part 1. The said combination process may be carried out by using methods such as dry mixing etc. that are commonly used in the current state of the art. Otilonium and excipients contained in Part 2 of the composition may be formulated by using methods such as dry mixing, dry granulation or wet granulation widely used in the current state of the art. Thereafter, Part 1 and Part 2 are compressed as a bilayer tablet.

As a result of studies, the inventors observed that no decrease of dissolution of otilonium bromide in the bilayer tablet that are prepared and compressed in accordance with present invention. Dissolution studies of otilonium included in the present composition are given in Table 4.

The present invention will be described in more detail by way of the following examples, but scope of the present invention is not limited thereto.

**Example 1**

| **Ingredients** | **Quantity** |
|---|---|
| ***Part 1*** | |
| Simethicone | 80 mg |
| Colloidal silicon dioxide | 50 mg |
| Silicified microcrystalline cellulose | 300 mg |
| Copovidone | 10 mg |
| Magnesium stearate | 10 mg |

| ***Part 2*** | |
|---|---|
| Otilonium bromide | 40 mg |
| Lactose | 150 mg |
| Crospovidone | 10 mg |
| Colloidal silicon dioxide | 1 mg |
| Magnesium stearate | 2.5 mg |
| **Total** | **653.5 mg** |

Simethicone is absorbed on colloidal silicon dioxide. Then, the final simethicone mixture is obtained by mixing silicified microcrystalline cellulose, copovidone and finally magnesium stearate. The final otilonium mixture is obtained by mixing lactose, copovidone, colloidal silicon dioxide and finally magnesium stearate. The obtained mixtures are compressed as bilayer tablets and optionally coated with a suitable coating material.

All solid dosage forms that may be manufactured based on the ideas and doctrines relate to the formulation of the present invention are disclosed by object of the invention.

**Table 1 - Stability study for the simethicone and otilonium formulation containing a basic agent**

| | **25°C/605 RH** | | | **30°C/65% RH** | | | **40°C/75% RH** | | |
|---|---|---|---|---|---|---|---|---|---|
| | **Initial** | **3^{rd} Months** | **6^{th} Months** | **Initial** | **3^{rd} Months** | **6^{th} Months** | **Initial** | **3^{rd} Months** | **6^{th} Months** |
| ***Maximum Unspecified** Impurities Max. 0.2 %* | 0.072 (RRT:0.60) | 0.164 (RRT:2.91) | 0.341* (RRT:2.93) | 0.072 (RRT:0.60) | 0.321* (RRT:2.91) | 0.632* (RRT:2.93) | 0.072 (RRT:0.60) | 1.195* (RRT:2.91) | 1.860* (RRT:2.93) |
| ***Total Impurities** Max. 2.0%* | 0.091 | 0.276 | 0.447 | 0.091 | 0.434 | 0.740 | 0.091 | 1.309 | 1.986 |
| *RH: Relative humidity* | | | | | | | | | |
| ** Out of Limit finding* | | | | | | | | | |

**Table 2 - Stability study for simethicone and otilonium - Assay**

| | **Initial** | **3^{rd} Months 25°C/60% RH** | **3^{rd} Months 30°C/65% RH** | **3^{rd} Months 40°C/75 % RH** | **6^{th} Months 25°C/60% RH** | **6^{th} Months 30°C/65% RH** | **6^{th} Months 40°C/75% RH** |
|---|---|---|---|---|---|---|---|
| **% Simethicone** | 101.1 | 94.5 | 92.9 | 93.6 | 96.4 | 93.9 | 93.0 |
| **% Otilonium** | 99.2 | 99.1 | 100.1 | 98.2 | 100.0 | 101.6 | 99.4 |
| **Maximum Unspecified Impurities** | 0.01 (RRT 1.39) | ND | <LOQ (RRT 0.61) | <LOQ (RRT 0.61) | 0.01 (RRT 1.39) | 0.01 (RRT 1.39) | 0.05 (RRT 3.25) |
| **Total Impurities** | 0.01 | ND | <LOQ | <LOQ | 0.02 | 0.01 | 0.06 |
| *LOQ: Limit of quantification* | | | | | | | |
| *TE: Not Detected* | | | | | | | |
| *RRT: Relative retention time* | | | | | | | |

**Table 3 - Effect of Simethicone on the Dissolution Profile of Otilonium Bromide**

| **Time (min)** | **Amount of Otilonium Bromide** |
|---|---|
| **15^{th} minutes (Otilonium bromide)** | 103.4 |
| **30^{th} minutes (Otilonium bromide+ Simethicone)** | 89.5 |
| **60^{th} minutes (Otilonium bromide+ Simethicone)** | 85.4 |
| **90^{th} minutes (Otilonium bromide+ Simethicone)** | 83.0 |

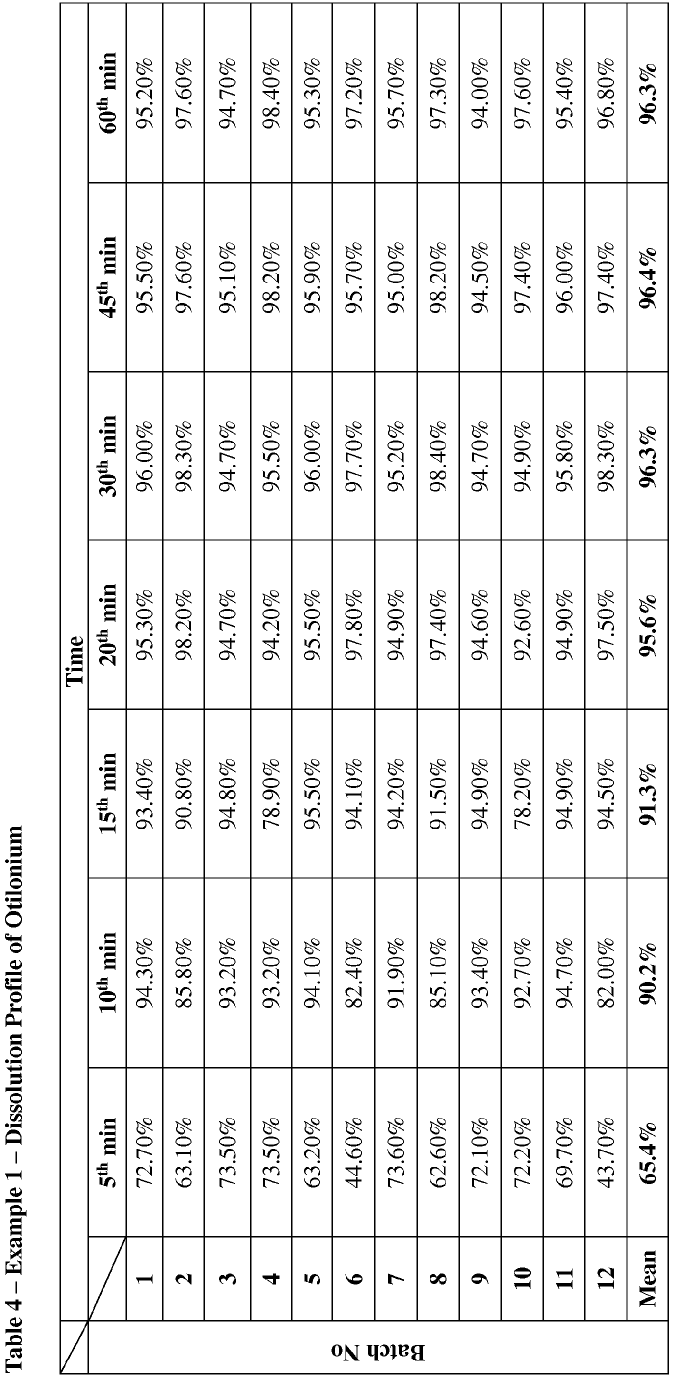

## Claims

1. A pharmaceutical composition in the form of a bilayer tablet comprising of a first part containing simethicone absorbed on colloidal silicon dioxide and a second part containing otilonium or pharmaceutically acceptable salts thereof, wherein the simethicone is absorbed on colloidal silicone dioxide and wherein the composition does not contain any basic agent.

2. A pharmaceutical composition according to Claim 1, wherein the composition further comprises microcrystalline cellulose or silicified microcrystalline cellulose.

3. A pharmaceutical composition according to Claim 1, wherein the composition comprises from 10 mg to 1000 mg of simethicone.

4. A pharmaceutical composition according to Claim 1, wherein the composition comprises from 10 mg to 500 mg of otilonium or pharmaceutically acceptable salt thereof.

5. A pharmaceutical composition according to Claiml, wherein the composition further comprise at least one pharmaceutically acceptable excipient selected from a group consisting of filler, binder, disintegrant, glidant, lubricant.

6. A pharmaceutical composition according to Claim 5, wherein the filler is selected from a group consisting of microcrystalline cellulose, lactose, maltose, mannitol, sugar.

7. A pharmaceutical composition according to Claim 6, wherein the filler is lactose.

8. A pharmaceutical composition according to Claim 5, wherein the binder is selected from the group consisting of polyvinylpyrrolidone, copovidone, cellulose, ethyl cellulose, hydroxymethyl cellulose, hydroxypropyl methyl cellulose, lactose, maltose and mannitol.

9. A pharmaceutical composition according to Claim 8, wherein the binder is copovidone.

10. A pharmaceutical according to Claim 5, wherein the disintegrant is selected from the group consisting of carboxymethyl cellulose, hydroxypropyl cellulose, hydroxypropyl methylcellulose, polyvinylpyrrolidone, crospovidone, microcrystalline cellulose, sodium starch glycolate, alginic acid and sodium alginate.

11. A pharmaceutical composition according to Claim 10, wherein the disintegrant is crospovidone.

12. A pharmaceutical composition according to Claim 5, wherein the glidant is selected from the group consisting of magnesium stearate, colloidal silicon dioxide, starch and talc.

13. A pharmaceutical composition according to Claim 5, wherein the lubricant is selected from the group consisting of stearic acid, magnesium stearat, talc, polyethylene glycol, sodium stearyl fumarate, sodium lauryl sulfate.

14. A pharmaceutical composition according to claim 1 in the form of a bilayer tablet, wherein the bilayer tablet comprises a first part and a second part, wherein the first part containing simethicone absorbed on colloidal silicon dioxide, microcrystalline cellulose or silicified microcrystalline cellulose, binder, glidant and lubricant; and the second part containing otilonium bromide, filler, disintegrant, binder, glidant and lubricant.

## Patentansprüche

1. Pharmazeutische Zusammensetzung in Form einer zweischichtigen Tablette; umfassend einen ersten Teil, der auf kolloidalem Siliziumdioxid absorbiertes Simethicon enthält; und einen zweiten Teil, der Otilonium oder pharmazeutisch verträgliche Salze enthält, wobei; das Simethicon wird an kolloidalem Silikondioxid absorbiert; wobei die Zusammensetzung kein basisches Mittel enthält.

2. Pharmazeutische Zusammensetzung nach Anspruch 1; wobei die Zusammensetzung ferner mikrokristalline Cellulose oder verkieselte mikrokristalline Cellulose umfasst.

3. Pharmazeutische Zusammensetzung nach Anspruch 1; wobei die Zusammensetzung 10 mg bis 1000 mg Simethicon umfasst.

4. Pharmazeutische Zusammensetzung nach Anspruch 1; wobei die Zusammensetzung 10 mg bis 500 mg Otilonium oder ein pharmazeutisch verträgliches Salz davon umfasst.

5. Pharmazeutische Zusammensetzung nach Anspruch 1; wobei die Zusammensetzung ferner mindestens einen pharmazeutisch verträglichen Exzipienten umfasst, ausgewählt aus einer Gruppe bestehend aus Füllstoff, Bindemittel, Sprengmittel, Gleitmittel, Schmiermittel.

6. Pharmazeutische Zusammensetzung nach Anspruch 5; wobei der Füllstoff ausgewählt ist aus einer Gruppe bestehend aus mikrokristalliner Cellulose, Lactose, Maltose, Mannit, Zucker.

7. Pharmazeutische Zusammensetzung nach Anspruch 6; wobei der Füllstoff Lactose ist.

8. Pharmazeutische Zusammensetzung nach Anspruch 5; wobei das Bindemittel ausgewählt ist aus der Gruppe bestehend aus Polyvinylpyrrolidon, Copovidon, Cellulose, Ethylcellulose, Hydroxymethylcellulose, Hydroxypropylmethylcellulose, Lactose, Maltose und Mannit.

9. Pharmazeutische Zusammensetzung nach Anspruch 8; wobei das Bindemittel Copovidon ist.

10. Arzneimittel nach Anspruch 5; wobei das Sprengmittel ausgewählt ist aus der Gruppe bestehend aus Carboxymethylcellulose, Hydroxypropylcellulose, Hydroxypropylmethylcellulose, Polyvinylpyrrolidon, Crospovidon, mikrokristalline Cellulose, Natriumstärkeglycolat, Alginsäure und Natriumalginat.

11. Pharmazeutische Zusammensetzung nach Anspruch 10; wobei das Sprengmittel Crospovidon ist.

12. Pharmazeutische Zusammensetzung nach Anspruch 5; wobei das Gleitmittel ausgewählt ist aus der Gruppe bestehend aus Magnesiumstearat, kolloidalem Siliziumdioxid, Stärke und Talk.

13. Pharmazeutische Zusammensetzung nach Anspruch 5; wobei das Schmiermittel ausgewählt ist aus der Gruppe bestehend aus Stearinsäure, Magnesiumstearat, Talkum, Polyethylenglycol, Natriumstearylfumarat, Natriumlaurylsulfat.

14. Pharmazeutische Zusammensetzung nach Anspruch 1 in Form einer zweischichtigen Tablette; wobei die zweischichtige Tablette einen ersten Teil und einen zweiten Teil umfasst, wobei der erste Teil Simethicon, absorbiert auf kolloidalem Siliziumdioxid, mikrokristalline Cellulose oder verkieselte mikrokristalline Cellulose, Bindemittel, Gleitmittel und Schmiermittel enthält; und der zweite Teil enthält Otiloniumbromid, Füllstoff, Sprengmittel, Bindemittel, Gleitmittel und Schmiermittel.

## Revendications

1. Composition pharmaceutique sous la forme d'un comprimé bicouche comprenant une première partie contenant de la siméthicone absorbée sur du dioxyde de silicium colloïdal et une seconde partie contenant de l'otilonium ou des sels pharmaceutiquement acceptables de celui-ci, dans laquelle la siméthicone est absorbée sur du dioxyde de silicone colloïdal et dans laquelle la composition ne contient pas n'importe quel agent de base.

2. Composition pharmaceutique selon la revendication 1, dans laquelle la composition comprend en outre de la cellulose microcristalline ou de la cellulose microcristalline silicifiée.

3. Composition pharmaceutique selon la revendication 1, dans laquelle la composition comprend de 10 mg à 1000 mg de siméthicone.

4. Composition pharmaceutique selon la revendication 1, dans laquelle la composition comprend de 10 mg à 500 mg d'otilonium ou de sel pharmaceutiquement acceptable.

5. Composition pharmaceutique selon la revendication 1, dans laquelle la composition comprend en outre au moins un excipient pharmaceutiquement acceptable choisi dans un groupe constitué d'une charge, d'un liant, d'un délitant, d'un glissant, d'un lubrifiant.

6. Composition pharmaceutique selon la revendication 5, dans laquelle la charge est choisie dans un groupe constitué de cellulose microcristalline, lactose, maltose, mannitol, sucre.

7. Composition pharmaceutique selon la revendication 6, dans laquelle la charge est du lactose.

8. Composition pharmaceutique selon la revendication 5, dans laquelle le liant est choisi dans le groupe constitué par la polyvinylpyrrolidone, la copovidone, la cellulose, l'éthylcellulose, l'hydroxyméthylcellulose, l'hydroxypropylméthylcellulose, le lactose, le maltose et le mannitol.

9. Composition pharmaceutique selon la revendication 8, dans laquelle le liant est la copovidone.

10. Composition pharmaceutique selon la revendication 5, dans lequel le délitant est choisi dans le groupe constitué de la carboxyméthylcellulose, l'hydroxypropylcellulose, l'hydroxypropylméthylcellulose, la polyvinylpyrrolidone, la crospovidone, la cellulose microcristalline, le glycolate d'amidon sodique, l'acide alginique et alginate de sodium.

11. Composition pharmaceutique selon la revendication 10, dans laquelle le délitant est la crospovidone.

12. Composition pharmaceutique selon la revendication 5, dans laquelle le glissant est choisi dans le groupe constitué du stéarate de magnésium, du dioxyde de silicium colloïdal, de l'amidon et du talc.

13. Composition pharmaceutique selon la revendication 5, dans laquelle le lubrifiant est choisi dans le groupe constitué par l'acide stéarique, le stéarate de magnésium, le talc, le polyéthylèneglycol, le stéarylfumarate de sodium, le laurylsulfate de sodium.

14. Composition pharmaceutique selon la revendication 1 sous la forme d'un comprimé bicouche, dans laquelle le comprimé bicouche comprend une première partie et une seconde partie, dans laquelle la première partie contenant de la siméthicone absorbée sur du dioxyde de silicium colloïdal, de la cellulose microcristalline ou de la cellulose microcristalline silicifiée, un liant, un agent glissant et lubrifiant; et la seconde partie contenant du bromure d'otilonium, une charge, un délitant, un liant, un glissant et un lubrifiant.
